(19) Europäisches Patentamt
European Patent Office
Office européen des brevets

(11) EP 3 730 480 B1

(12) EUROPEAN PATENT SPECIFICATION

(45) Date of publication and mention of the grant of the patent:
**29.05.2024 Bulletin 2024/22**

(21) Application number: **17935098.8**

(22) Date of filing: **27.12.2017**

(51) International Patent Classification (IPC):
***C07C 407/00*** (2006.01) ***C07D 301/19*** (2006.01)
***C07C 409/08*** (2006.01) ***C07D 303/04*** (2006.01)

(52) Cooperative Patent Classification (CPC):
(C-Sets available)
**C07C 407/00; C07D 301/19; C07D 303/04;**
Y02P 20/52 (Cont.)

(86) International application number:
**PCT/CN2017/118873**

(87) International publication number:
**WO 2019/119489 (27.06.2019 Gazette 2019/26)**

# (54) METHOD OF PREPARING ETHYLBENZENE HYDROPEROXIDE BY LIQUID-PHASE PEROXIDATION OF ETHYLBENZENE AND PREPARATION METHOD OF PROPYLENE OXIDE

VERFAHREN ZUR HERSTELLUNG VON ETHYLBENZOLHYDROPEROXID DURCH FLÜSSIGPHASENPEROXIDATION VON ETHYLBENZOL UND VERFAHREN ZUR HERSTELLUNG VON PROPYLENOXID

PROCÉDÉ DE PRÉPARATION D'HYDROPEROXYDE D'ÉTHYLBENZÈNE PAR PEROXYDATION EN PHASE LIQUIDE D'ÉTHYLBENZÈNE ET PROCÉDÉ DE PRÉPARATION D'OXYDE DE PROPYLÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2017 CN 201711390431**

(43) Date of publication of application:
**28.10.2020 Bulletin 2020/44**

(73) Proprietor: **Wanhua Chemical Group Co., Ltd.**
**Yantai, Shandong 264002 (CN)**

(72) Inventors:
- **LIU, Peng**
  **Yantai, Shandong 264002 (CN)**
- **DAI, Hongtao**
  **Yantai, Shandong 264002 (CN)**
- **SUN, Qinhe**
  **Yantai, Shandong 264002 (CN)**
- **LIU, Ruochen**
  **Yantai, Shandong 264002 (CN)**
- **SUN, Xican**
  **Yantai, Shandong 264002 (CN)**
- **LI, Xingwei**
  **Yantai, Shandong 264002 (CN)**
- **LANG, Xiaojun**
  **Yantai, Shandong 264002 (CN)**
- **YU, Tianyong**
  **Yantai, Shandong 264002 (CN)**

(74) Representative: **De Gregori, Antonella et al**
**BIRD & BIRD SOCIETA TRA AVVOCATI S.R.L.**
**Via Porlezza, 12**
**20123 Milano (IT)**

(56) References cited:
**WO-A1-2015/101641** **WO-A1-2015/101641**
**CN-A- 101 538 020** **CN-A- 101 851 187**
**CN-A- 106 554 298** **GB-A- 681 990**
**JP-A- H0 459 756**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE CAPLUS [Online] CHEMICAL ABSTRACTS SERVICE, COLUMBUS, OHIO, US; POKORSKA, Z; RISZER, R; SPADLO, M: "Liquid phase oxidation of ethylbenzene by molecular oxygen to obtain the hydroperoxide", XP002804229, retrieved from STN Database accession no. 1973:159125 & POKORSKA, Z; RISZER, R; SPADLO, M: ZESZYTY NAUKOWE - INSTYTUT CIEZKIEJ SYNTEZY ORGANICZEJ, vol. 4, no. 19, 31 December 1972 (1972-12-31),**

(52) Cooperative Patent Classification (CPC): (Cont.)

C-Sets
**C07C 407/00, C07C 409/08**

## Description

### Field of the Invention

The invention relates to a method for preparing ethylbenzene hydroperoxide by liquid phase peroxidation of ethylbenzene and a preparation method of propylene oxide, in particular to a method for preparing ethylbenzene hydroperoxide by adding basic organic compound to promote peroxidation of ethylbenzene and a method of further preparing propylene oxide.

### Background of the Invention

Propylene oxide (PO) and styrene (SM) co-production method (also known as PO/SM method) is considered to be one of the best processes for industrial production of propylene oxide due to its low pollution and low energy consumption. The process mainly includes three steps, namely:

1. excess liquid ethylbenzene is contacted with oxygen in the air or a mixed gas containing oxygen to form ethylbenzene hydroperoxide (EBHP);
2. under high pressure, EBHP is reacted with excess liquid phase propylene to form propylene oxide and phenylmethyl alcohol (PMA); and
3. after the propylene oxide is removed from the excess propylene, the residual liquid containing PMA is introduced into the dehydration reactor to form styrene monomer (SM) by dehydration reaction.

The reaction route can be expressed by the following formula:

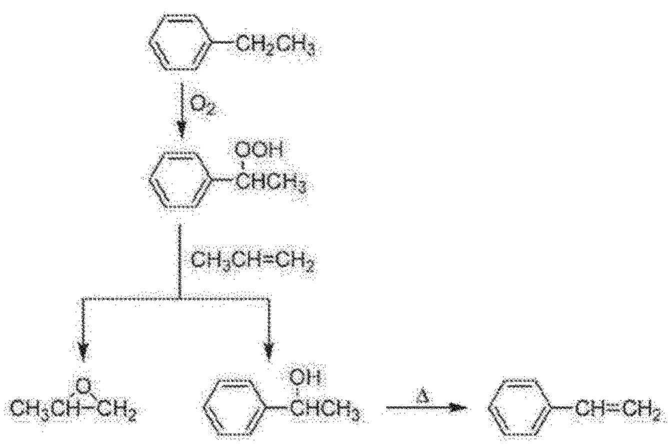

It can be seen from the above steps that in the process of ethylbenzene peroxidation, the conversion and selectivity of EBHP are directly related to the yield of PO. Therefore, in general, the annual production capacity of PO/SM devices depends on the effect of ethylbenzene peroxidation. However, the selectivity of EBHP as an unstable peroxide has a tendency to decrease with the increase of conversion under normal operating conditions. This inverse relationship between the selectivity and conversion fundamentally limits the stability and economy of the PO/SM device.

Here, based on the number of moles, the conversion of ethylbenzene peroxidation is defined as:

$$\frac{\text{molar number of ethylbenzene reacted}}{\text{molar number of ethylbenzene feed}} \times 100\%$$

The selectivity of ethylbenzene peroxidation, also based on the number of moles, is defined as:

$$\frac{\text{molar number of ethylbenzene hydroperoxide produced}}{\text{molar number of ethylbenzene reacted}} \times 100\%$$

At present, the low ethylbenzene conversion (less than 10%) and relatively low target product EBHP selectivity (less than 90%) in the industrial process are the specific manifestations of the above inverse relationship, and also the

main problem that restricts the large-scale PO/SM process.

The specific reaction of ethylbenzene peroxidation is showed as the formula below: EBHP is also decomposed into PMA and phenyl methyl ketone (PMK) while EBHP is formed. If the selectivity of the reaction is too low, the ratio of the final product PO and SM in the PO/SM device will be unbalanced, the load on the subsequent devices will be increased, and the production efficiency of PO will be lowered.

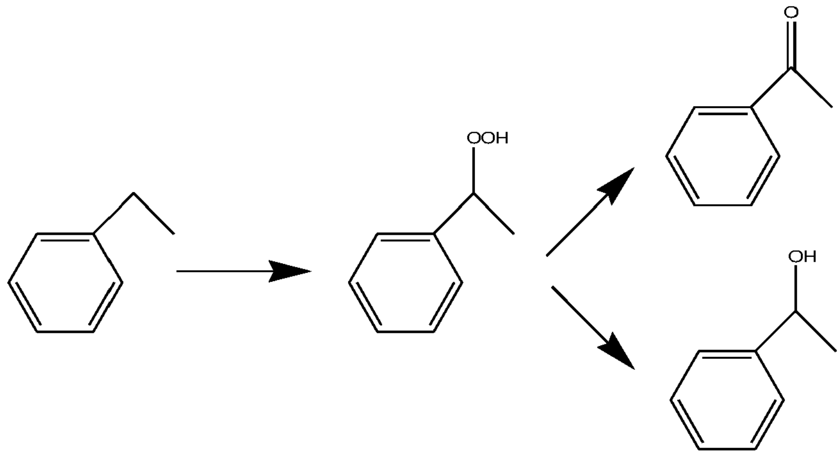

At present, many attempts have been made to additionally add a small amount of "catalyst", "inhibitor", and "stabilizer" to the ethylbenzene peroxidation reaction, in order to increase the selectivity of the peroxidation reaction. US2798096 and US3816540 have disclosed a method for increasing the selectivity of the reaction by adding pyrophosphate to the ethylbenzene peroxidation reaction; and US2820832 relates to a method for stabilizing ethylbenzene peroxide reaction by using metal salt of copper or silver. In US4262143, Halcon Corporation discloses a technique for improving the selectivity of ethylbenzene peroxidation using sodium hydroxide/ potassium hydroxide or corresponding metal salts.

Although the above techniques are partially applied to the current industrial production, some special metal salts are additionally introduced. These metal salts may precipitate during the subsequent epoxidation and deposit on the surface of the epoxidation catalyst, causing rapid deactivation of the catalyst, thereby affecting the stable operation of the device. In order to avoid the deposition of metal salts, some PO/SM processes have increased the corresponding water washing procedure to remove the corresponding salts as much as possible before the epoxidation reaction. However, such processes often result in additional EBHP losses, increasing the risk of PMA emulsification in the aqueous phase and thereby destabilizing the operation.

CHEMICAL ABSTRACTS ("Liquid phase oxidation of ethylbenzene by molecular oxygen to obtain the hydroperoxide", 1 December 1973 (1973-12-01), Columbus, Ohio, US; abstract no.: 1973:159125, POKORSKA, Z; RISZER, R; SPADLO, M) discloses a process for the preparation of ethylbenzene hydroperoxide via liquid-phase peroxidation by oxygen, wherein pyridine is used as an additive.

WO2015/101641A1 discloses a process for preparing an alkylene oxide comprising preparing an alkyl phenyl hydroperoxide and contacting the alkyl phenyl hydroperoxide with an alkene in an epoxidation reaction to obtain an alkylene oxide and an alkyl phenyl alcohol.

In view of the above technical problems in the ethylbenzene peroxidation reaction, it is necessary to find a new method to improve the selectivity of EBHP in the peroxidation reaction.

## Summary of the Invention

The object of the present invention is to provide a method for improving the liquid phase peroxidation of ethylbenzene to prepare ethylbenzene hydroperoxide, and the method does not adversely affect the subsequent epoxidation reaction, and does not add additional devices.

In order to achieve the above object, the technical solution adopted by the present invention is as follows.

A first object of the present invention is to provide a process for preparing ethylbenzene hydroperoxide (EBHP) by liquid phase peroxidation of ethylbenzene, comprising: contacting and reacting a gas containing oxygen molecule and a liquid phase of ethylbenzene in the presence of an organic basic substance to form ethylbenzene hydroperoxide-wherein the organic basic substance is one or more selected from the group consisting of guanidine compounds.

The added organic basic substance is soluble in the reaction system mixture (mainly ethylbenzene, EBHP, PMA, PMK, etc. and is selected among guanidine compounds, such as guanidine nitrate, guanidine carbonate, etc.,

and more preferably guanidine carbonate.

According to the process of the present invention, the organic basic substance is added in an amount of from 0.1 to 200 ppm, preferably from 0.2 to 100 ppm, more preferably from 0.5 to 50 ppm, based on the mass of the ethylbenzene. A small amount of organic basic substance added to the reaction system increases EBHP selectivity.

According to the process of the present invention, the reaction temperature of ethylbenzene with the oxygen-containing gas is generally in the range of from 100 to 220 °C, preferably from 100 to 170 °C, more preferably from 120 to 160 °C. When the temperature is lower than 120 °C, the reaction rate is too low. When the temperature is higher than 160 °C, the reaction rate of the side reaction is significantly accelerated, thereby lowering the selectivity of ethylbenzene hydroperoxide. To further increase the selectivity of the hydroperoxide, the peroxidation reaction can be subjected to a stepwise cooling operation to minimize the formation of by-products. Although the corresponding control strategy may be slightly more complicated, the overall reaction time can be significantly shortened. Therefore, the peroxidation reaction temperature is controlled to 130-160 °C in the early stage of the reaction, and reduced to 120-150 °C in the later stage, which will facilitate the improvement of the overall efficiency of the device.

According to the process of the present invention, the reaction pressure of ethylbenzene with the oxygen-containing gas is generally from 0 to 20 barG, preferably from 0.5 to 14 barG, more preferably from 1 to 8 barG. The amount of the oxygen-containing gas added can be determined by those skilled in the art based on the scale of the apparatus as well as the operating temperature, pressure, and desired tail oxygen content (oxygen content in the tail gas).

According to the method of the present invention, the reaction time of ethylbenzene and the oxygen-containing gas is generally 0.5-20 hours, and the appropriate reaction time should be selected according to the reaction temperature and the partial pressure of oxygen. When the reaction temperature is 130-160 °C, the reaction time is preferably 0.5 -6 hours.

In the present invention, the oxygen-containing gas may be selected from any gas containing oxygen molecules such as air or oxygen.

A second object of the present invention is to provide a process for preparing propylene oxide, comprising:

(A) a gas containing oxygen molecules is contacted and reacted with liquid ethylbenzene in the presence of an organic basic substance to obtain a peroxidation reaction solution containing ethylbenzene hydroperoxide;
(B) a part of the unreacted ethylbenzene is removed from the peroxidation reaction solution obtained in step (A), preferably concentrated to an ethylbenzene hydroperoxide concentration of 20-50% by weight, and then an epoxidation reaction is performed with propylene.

The partial removal of unreacted ethylbenzene can be carried out by distillation, and the distillation can be carried out at 0-80 KPaA, 80-110 °C, and distilled to an EBHP concentration of, for example, 20-50 wt%, and the distillation time is generally 5- 60 minutes.

As described above, the added organic alkaline substance can be dissolved in the reaction system mixture (mainly ethylbenzene, EBHP, PMA, PMK, etc. and is selected among guanidine compounds, such as guanidine nitrate, guanidine carbonate, etc., and more preferably guanidine carbonate.

As described above, the organic basic substance is added in an amount of 0.1 to 200 ppm, preferably 0.2 to 100 ppm, more preferably 0.5 to 50 ppm based on the mass of ethylbenzene.

As described above, the reaction temperature of ethylbenzene with an oxygen-containing gas is generally in the range of 100 to 220 °C, preferably 100 to 170 °C, more preferably 120 to 160°C. When the temperature is lower than 120 °C, the reaction rate is too low. When the temperature is higher than 160 °C, the reaction rate of side reaction is significantly accelerated, thereby lowering the selectivity of ethylbenzene hydroperoxide. To further increase the selectivity of ethylbenzene hydroperoxide, the peroxidation reaction can be subjected to a stepwise cooling operation to minimize the formation of by-products. Although the corresponding control strategy may be slightly more complicated, the overall reaction time can be significantly shortened. Therefore, the peroxidation reaction temperature is controlled to 130-160 °C in the early stage of the reaction, and reduced to 120-150 °C in the later stage, which will facilitate the improvement of the overall efficiency of the device.

As described above, the reaction pressure of ethylbenzene with an oxygen-containing gas is generally from 0 to 20 barG, preferably from 0.5 to 14 barG, more preferably from 1 to 8 barG.

As described above, the reaction time of ethylbenzene and the oxygen-containing gas is generally 0.5-20 hours, and the appropriate reaction time should be selected according to the reaction temperature and the partial pressure of oxygen. When the reaction temperature is 130-160 °C, the reaction time is preferably 0.5-6 hour. The gas containing oxygen molecules may be selected from any gas containing oxygen molecules such as air, oxygen, diluted oxygen, and the like.

According to the process of the present invention, the epoxidation reaction is carried out in the presence of a catalyst, and the reaction temperature is usually from 20 to 220 °C, preferably from 30 to 160 °C, more preferably from 50 to 130 °C; the molar ratio of propylene to EBHP in the feed is generally 2:1-10:1, preferably 3:1-8:1; the epoxidation

reaction pressure is such that the reaction system is maintained in a full liquid phase state, and the pressure is generally from 5 to 70 barG, preferably from 30 to 60 barG.

According to the method of the present invention, the epoxidation catalyst may comprise elements such as Ti, V, Cr, Mo, W, etc., and the catalysts disclosed in the patents CN201610021367, CN201510639697, CN201610601021, etc. may be employed.

According to the method of the present invention, the organic basic substance added during the peroxidation reaction does not introduce a metal base or a salt, thereby avoiding the influence of the deposition of the metal base or salt on the surface of the catalyst on the activity of the catalyst during the epoxidation process, and thus the influence on the efficiency of the epoxidation reaction.

## Brief Description of the Drawing

Figure 1 is a graph showing the relationship between EBHP concentration wt% and EBHP selectivity mol% in the examples.

## The Mode of Carrying Out the Invention

The invention will be further described in detail below with reference to examples.

In the following examples, the apparatus used was equipped with a 500 ml reaction kettle, which was made of 316 stainless steel. The reaction kettle was equipped with a stirrer and a gas distributor to ensure uniform bubble dispersion and mass transfer during the experiment. At the same time, it was equipped with temperature sensor, external heating jacket, internal cooling coil, cooling oil storage tank and oil pump to precisely control the temperature inside the reactor. It was also equipped with air and nitrogen bottled air supply, intake valve, safety valve, pressure gauge, gas phase condenser, back pressure valve etc. The intake air flow rate was regulated by a needle valve, and the oxidation tail gas was discharged through the outlet, and the organic matter in the exhaust gas was recovered into the reaction kettle by condensation, and the pressure in the reaction kettle was controlled by the exhaust gas back pressure valve.

The sampling system was a two-valve sampler that includes a sampling tube, sample tube, cooling jacket, front and rear valves, solvent and nitrogen storage tanks, solvent, nitrogen purge channels etc. The purpose of using solvent and nitrogen purge was to ensure that there are no residual impurities in the sample tube before each sampling to avoid sample distortion.

Reference example 1

250 ml of ethylbenzene was weighed into the reaction vessel and sealed. The air in the reaction vessel was replaced by nitrogen gas repeatedly, and pressurized to about 1 barG, the reactor inlet was closed, and the back pressure valve at the reactor exhaust gas outlet was adjusted to a closed state. The reactor mechanical agitation, electric heating and circulating water in exhaust gas condensation pipe were turn on, the computer temperature control program was started, and the reactor temperature was set to 150 °C, the stirring speed to 800 r/min. When the temperature in the reaction kettle reached the set value, the air intake valve was opened, the reaction was started and the time was recorded, and a first sample was simultaneously sampled. Thereafter, samples were taken every 5-20 minutes, and a solvent and nitrogen purge were required before and after each sampling, and the total reaction time was 3.5 hr. The data during the experiment (temperature in the kettle, air flow, concentration of $O_2$, CO and $CO_2$ in the exhaust gas, etc.) were collected and recorded in real time by a computer. According to the analysis results, EBHP selectivity under different EBHP concentrations was obtained. The results were shown in the following table:

| EBHP concentration wt% | 1.9 | 3.3 | 4.3 | 5.8 | 7.7 | 9.3 | 10.8 |
|---|---|---|---|---|---|---|---|
| EBHP selectivity mol% | 92.1 | 89.0 | 86.6 | 83.2 | 78.7 | 74.2 | 70.7 |

Reference example 2

250 ml of ethylbenzene was weighed, 10 ppm (based on ethylbenzene mass) of sodium hydroxide was added thereto and formulated as a solution, and the above solution was added to a reaction vessel and sealed. The other experimental procedures were the same as in Example 1. According to the analysis results, EBHP selectivity under different EBHP concentrations was obtained. The results were shown in the following table:

| EBHP concentration wt% | 2.2 | 3.6 | 5.4 | 6.8 | 8.1 | 9.5 | 11.2 |
|---|---|---|---|---|---|---|---|

(continued)

| | | | | | | | |
|---|---|---|---|---|---|---|---|
| EBHP selectivity mol% | 92.7 | 90.1 | 85.7 | 82.4 | 78.9 | 76.1 | 72.7 |

Example 3

250 ml of ethylbenzene was weighed, 10 ppm (based on ethylbenzene mass) of guanidine carbonate was added and formulated as a solution, and the above solution was added to a reaction vessel and sealed. The other experimental procedures were the same as in Example 1. According to the analysis results, EBHP selectivity under different EBHP concentrations can be obtained. The results were shown in the following table:

| EBHP concentration wt% | 2.7 | 4.1 | 5.5 | 6.8 | 8.1 | 9.4 | 10.3 |
|---|---|---|---|---|---|---|---|
| EBHP selectivity mol% | 93.1 | 90.2 | 86.5 | 83.4 | 80.1 | 78.1 | 76.6 |

Example 4 (not according to the invention)

250 ml of ethylbenzene was weighed, 10 ppm (based on ethylbenzene mass) of 4-methylpyridine was added and formulated as a solution, and the above solution was added to a reaction vessel and sealed. The other experimental procedures were the same as in Example 1. According to the analysis results, EBHP selectivity under different EBHP concentrations was obtained. The results were shown in the following table:

| EBHP concentration wt% | 1.5 | 2.8 | 4.6 | 6.7 | 8.3 | 9.7 | 10.9 |
|---|---|---|---|---|---|---|---|
| EBHP selectivity mol% | 94.3 | 92.1 | 87.8 | 82.4 | 79.1 | 76.4 | 73.9 |

Comparing the results of Examples 2, 3, and 4 with Example 1 (see Figure 1), it can be seen that the addition of a basic substance (whether sodium hydroxide, guanidine carbonate or 4-methylpyridine) to the reaction system is advantageous to improve the selectivity of EBHP, in which the effect of guanidine carbonate is more significant.

Example 5

Part of the unreacted ethylbenzene was separated from the peroxidation reaction liquids in Examples 1-4 by distillation (distillation pressure: 50 KPaA, temperature: 105 °C, distillation time: 20 minutes) to increase the EBHP concentration to 20% by weight. The above concentrate was epoxidized with propylene (propylene to EBHP molar ratio of 8:1) in an adiabatic fixed bed. The fixed bed reactor used was 20 mm in diameter and 1500 mm in length, in which the catalyst disclosed in Example 1 of the patent CN106334583A was charged. The feed flow rate was 360 ml/min, the reactor pressure was 60 barG, and the inlet temperature was 80°C. The outlet temperature was gradually lowered from about 140 °C to about 135 °C due to a decrease in catalyst activity. After 45 consecutive days of experimentation, the results of using different peroxidation reaction solutions were compared as follows:

| reaction solution | EBHP conversion rate % | PO selectivity mol% |
|---|---|---|
| Reference example 1 | 92.1 | 88.4 |
| Reference example 2 | 87.6 | 87.2 |
| Example 3 | 92.5 | 88.3 |
| Example 4 | 92.0 | 88.4 |

The above results indicate that although the addition of sodium hydroxide or guanidine carbonate or 4-methylpyridine can increase the selectivity of EBHP during the peroxidation process, the conversion of EBHP and the selectivity of PO will be reduced by adding sodium hydroxide, while the conversion of EBHP and the selectivity of PO will not be affected by adding organic base (guanidine carbonate or 4-methylpyridine), and the effect of guanidine carbonate is more significant and its reaction effect is equivalent to that of Reference example 1 without adding any substance. Analysis shows that during the epoxidation process, the inorganic alkali metal deposits on the surface of the catalyst, resulting in a decrease in catalyst performance, while the organic base does not deposit on the catalyst surface.

## Claims

1. A method for preparing ethylbenzene hydroperoxide by liquid phase peroxidation of ethylbenzene, which comprises: contacting and reacting a gas containing oxygen molecules with liquid ethylbenzene in the presence of an organic basic substance to form ethylbenzene hydroperoxide, wherein the organic basic substance is one or more selected from the group consisting of guanidine compounds.

2. The method as claimed in claim 1 wherein the added organic basic substance contains no more than 20 carbon atoms and is capable of dissolving in the reaction system mixture.

3. The method as claimed in claim 1, wherein the organic basic substance is guanidine nitrate and/or guanidine carbonate.

4. The method as claimed in claim 1, wherein the organic basic substance is selected from guanidine carbonate.

5. The method as claimed in any one of claims 1 to 4, wherein the organic basic substance is added in an amount of 0.1 to 200 ppm, preferably 0.2 to 100 ppm, more preferably 0.5 to 50 ppm based on the mass of the ethylbenzene.

6. The method as claimed in any one of claims 1 to 5, wherein the reaction temperature of ethylbenzene with a gas containing oxygen molecules is from 100 to 220 °C, preferably from 100 to 170 °C, more preferably from 120 to 160 °C; the reaction pressure of ethylbenzene and the gas containing oxygen molecules is from 0 to 20 bar G, preferably from 0.5 to 14 bar G, more preferably from 1 to 8 bar G; and the reaction time of ethylbenzene with the gas containing oxygen molecules is from 0.5 to 20 hours.

7. The method as claimed in any one of claims 1 to 6, wherein the gas containing oxygen molecules is selected from the group consisting of air and oxygen.

8. A method for preparing propylene oxide, the method comprising:

   (A) a gas containing oxygen molecules is contacted and reacted with liquid ethylbenzene in the presence of an organic basic substance to obtain a peroxidation reaction solution containing ethylbenzene hydroperoxide;
   (B) a part of the unreacted ethylbenzene is removed from the peroxidation reaction solution obtained in step (A), preferably concentrated to an ethylbenzene hydroperoxide concentration of 20-50% by weight, and then an epoxidation reaction is performed with propylene, wherein the organic basic substance is one or more selected from the group consisting of guanidine compounds.

9. The method as claimed in claim 8, wherein the organic basic substance is guanidine nitrate and/or guanidine carbonate.

10. The method as claimed in claim 8, wherein the organic basic substance is selected from guanidine carbonate.

11. The method as claimed in claim 8 or 9, wherein the organic basic substance is added in an amount of from 0.1 to 200 ppm, preferably from 0.2 to 100 ppm, more preferably from 0.5 to 50 ppm, based on the mass of the ethylbenzene.

12. The method according to claim 8 or 9, wherein the reaction temperature of ethylbenzene with a gas containing oxygen molecules is from 100 to 220 °C, preferably from 100 to 170 °C, more preferably from 120 to 160 °C; the reaction pressure of ethylbenzene and the gas containing oxygen molecules is from 0 to 20 bar G, preferably from 0.5 to 14 bar G, more preferably from 1 to 8 bar G; and the reaction time of ethylbenzene with a gas containing oxygen molecules is from 0.5 to 20 hours.

13. The process as claimed in claim 8 or 9, wherein the epoxidation reaction is carried out in the presence of a catalyst at a temperature of from 20 to 220 °C, preferably from 30 to 160 °C, more preferably from 50 to 130 °C; and/or the molar ratio of propylene to ethylbenzene hydroperoxide in the feed is 2:1-10:1, preferably 3:1-8:1; the pressure of the epoxidation reaction is such that the reaction system is maintained in a full liquid phase state, and the pressure is preferably from 5 to 70 bar G, preferably from 30 to 60 bar G.

**Patentansprüche**

1. Verfahren zur Herstellung von Ethylbenzolhydroperoxid durch Flüssigphasenperoxidation von Ethylbenzol, umfassend: Inkontaktbringen und Umsetzen eines Gases, das Sauerstoffmoleküle enthält, mit flüssigem Ethylbenzol in Gegenwart einer organischen Grundsubstanz, um Ethylbenzolhydroperoxid zu bilden, wobei die organische Grundsubstanz eine oder mehrere ist, ausgewählt aus der Gruppe bestehend aus Guanidinverbindungen.

2. Verfahren nach Anspruch 1, wobei die zugegebene organische Grundsubstanz nicht mehr als 20 Kohlenstoffatome enthält und in der Lage ist, sich in dem Reaktionssystemgemisch zu lösen.

3. Verfahren nach Anspruch 1, wobei die organische Grundsubstanz Guanidinnitrat und/oder Guanidincarbonat ist.

4. Verfahren nach Anspruch 1, wobei die organische Grundsubstanz aus Guanidincarbonat ausgewählt ist.

5. Verfahren nach einem der Ansprüche 1 bis 4, wobei die organische Grundsubstanz in einer Menge von 0,1 bis 200 ppm, vorzugsweise 0,2 bis 100 ppm, besonders bevorzugt 0,5 bis 50 ppm, bezogen auf die Masse des Ethylbenzols, zugegeben wird.

6. Verfahren nach einem der Ansprüche 1 bis 5, wobei die Reaktionstemperatur von Ethylbenzol mit einem Gas, das Sauerstoffmoleküle enthält, von 100 bis 220 °C, vorzugsweise von 100 bis 170 °C, besonders bevorzugt von 120 bis 160 °C beträgt; der Reaktionsdruck von Ethylbenzol und dem Gas, das Sauerstoffmoleküle enthält, von 0 bis 20 bar G, vorzugsweise von 0,5 bis 14 bar G, besonders bevorzugt von 1 bis 8 bar G beträgt; und die Reaktionszeit von Ethylbenzol mit dem Gas , das Sauerstoffmoleküle enthält, von 0,5 bis 20 Stunden beträgt.

7. Verfahren nach einem der Ansprüche 1 bis 6, wobei das Gas, das Sauerstoffmoleküle enthält, ausgewählt ist aus der Gruppe bestehend aus Luft und Sauerstoff.

8. Verfahren zur Herstellung von Propylenoxid, wobei das Verfahren umfasst:

   (A) ein Gas, das Sauerstoffmoleküle enthält, wird mit flüssigem Ethylbenzol in Gegenwart einer organischen Grundsubstanz in Kontakt gebracht und umgesetzt, um eine Peroxidationsreaktionslösung zu erhalten, die Ethylbenzolhydroperoxid enthält;
   (B) ein Teil des nicht umgesetzten Ethylbenzols aus der in Schritt (A) erhaltenen Peroxidationsreaktionslösung wird entfernt, vorzugsweise auf eine Ethylbenzolhydroperoxidkonzentration von 20 - 50 Gew.-% konzentriert, und anschließend wird eine Epoxidationsreaktion mit Propylen durchgeführt, wobei die organische Grundsubstanz eine oder mehrere ist, ausgewählt aus der Gruppe bestehend aus Guanidinverbindungen.

9. Verfahren nach Anspruch 8, wobei die organische Grundsubstanz Guanidinnitrat, und/oder Guanidincarbonat ist.

10. Verfahren nach Anspruch 8, worin die organische Grundsubstanz aus Guanidincarbonat ausgewählt ist.

11. Verfahren nach Anspruch 8 oder 9, wobei die organische Grundsubstanz in einer Menge von 0,1 bis 200 ppm, vorzugsweise von 0,2 bis 100 ppm, besonders bevorzugt von 0,5 bis 50 ppm, bezogen auf die Masse des Ethylbenzols, zugegeben wird.

12. Verfahren nach Anspruch 8 oder 9, worin die Reaktionstemperatur von Ethylbenzol mit einem Gas , das Sauerstoffmoleküle enthält, von 100 bis 220 °C, vorzugsweise von 100 bis 170 °C, besonders bevorzugt von 120 bis 160 °C beträgt; der Reaktionsdruck von Ethylbenzol und dem Gas, das Sauerstoffmoleküle enthält, von 0 bis 20 bar G, vorzugsweise von 0,5 bis 14 bar G, besonders bevorzugt von 1 bis 8 bar G beträgt; und die Reaktionszeit von Ethylbenzol mit einem Gas, das Sauerstoffmoleküle enthält, 0,5 bis 20 Stunden beträgt.

13. Verfahren nach Anspruch 8 oder 9, wobei die Epoxidationsreaktion in Gegenwart von einem Katalysator bei einer Temperatur von 20 bis 220 °C, vorzugsweise von 30 bis 160 °C, besonders bevorzugt von 50 bis 130 °C durchgeführt wird; und/oder das Molverhältnis von Propylen zu Ethylbenzolhydroperoxid in der Beschickung 2:1 - 10:1, vorzugsweise 3:1 - 8:1 beträgt; der Druck der Epoxidationsreaktion derart ist, dass das Reaktionssystem in einem vollständigen Flüssigphasenzustand aufrechterhalten wird, und der Druck vorzugsweise von 5 bis 70 bar G, vorzugsweise von 30 bis 60 bar G beträgt.

## Revendications

1. Méthode pour préparer de l'hydroperoxyde d'éthylbenzène par peroxydation en phase liquide de l'éthylbenzène, qui comprend : mettre en contact et faire réagir un gaz contenant des molécules d'oxygène avec de l'éthylbenzène liquide en présence d'une substance organique basique pour former de l'hydroperoxyde d'éthylbenzène, dans laquelle la substance organique basique est l'une ou plusieurs des substances choisies dans le groupe constitué par les composés de guanidine.

2. Méthode telle que revendiquée dans la revendication 1, dans laquelle la substance organique basique ajoutée ne contient pas plus de 20 atomes de carbone et est capable de se dissoudre dans le mélange de système réactionnel.

3. Méthode telle que revendiquée dans la revendication 1, dans laquelle la substance organique basique est le nitrate de guanidine et/ou le carbonate de guanidine.

4. Méthode telle que revendiquée dans la revendication 1, dans laquelle la substance organique basique est choisie parmi le carbonate de guanidine.

5. Méthode telle que revendiquée dans l'une quelconque des revendications 1 à 4, dans laquelle la substance organique basique est ajoutée en une quantité de 0,1 à 200 ppm, préférentiellement de 0,2 à 100 ppm, plus préférentiellement de 0,5 à 50 ppm sur la base de la masse de l'éthylbenzène.

6. Méthode telle que revendiquée dans l'une quelconque des revendications 1 à 5, dans laquelle la température de réaction de l'éthylbenzène avec un gaz contenant des molécules d'oxygène est de 100 à 220 °C, préférentiellement de 100 à 170 °C, plus préférentiellement de 120 à 160 °C ; la pression de réaction de l'éthylbenzène et du gaz contenant des molécules d'oxygène est de 0 à 20 bars G, préférentiellement de 0,5 à 14 bars G, plus préférentiellement de 1 à 8 bars G ; et le temps de réaction de l'éthylbenzène avec le gaz contenant des molécules d'oxygène est de 0,5 à 20 heures.

7. Méthode telle que revendiquée dans l'une quelconque des revendications 1 à 6, dans laquelle le gaz contenant des molécules d'oxygène est choisi dans le groupe constitué de l'air et de l'oxygène.

8. Méthode pour préparer de l'oxyde de propylène, la méthode comprenant :

   (A) un gaz contenant des molécules d'oxygène est mis en contact et réagit avec de l'éthylbenzène liquide en présence d'une substance organique basique pour obtenir une solution de réaction de peroxydation contenant de l'hydroperoxyde d'éthylbenzène ;
   (B) une partie de l'éthylbenzène n'ayant pas réagi est éliminée de la solution de réaction de peroxydation obtenue à l'étape (A), préférentiellement concentrée à une concentration d'hydroperoxyde d'éthylbenzène de 20-50 % en poids, et puis une réaction d'époxydation est effectuée avec du propylène, dans laquelle la substance organique basique est l'une ou plusieurs des substances choisies parmi le groupe constitué par les composés de guanidine.

9. Méthode telle que revendiquée dans la revendication 8, dans laquelle la substance organique basique est le nitrate de guanidine et/ou le carbonate de guanidine.

10. Méthode telle que revendiquée dans la revendication 8, dans laquelle la substance organique basique est choisie parmi le carbonate de guanidine.

11. Méthode telle que revendiquée dans la revendication 8 ou 9, dans laquelle la substance organique basique est ajoutée en une quantité de 0,1 à 200 ppm, préférentiellement de 0,2 à 100 ppm, plus préférentiellement de 0,5 à 50 ppm, sur la base de la masse de l'éthylbenzène.

12. Méthode selon la revendication 8 ou 9, dans laquelle la température de réaction de l'éthylbenzène avec un gaz contenant des molécules d'oxygène est de 100 à 220 °C, préférentiellement de 100 à 170 °C, plus préférentiellement de 120 à 160 °C ; la pression de réaction de l'éthylbenzène et du gaz contenant des molécules d'oxygène est de 0 à 20 bars G, préférentiellement de 0,5 à 14 bars G, plus préférentiellement de 1 à 8 bars G ; et le temps de réaction de l'éthylbenzène avec un gaz contenant des molécules d'oxygène est de 0,5 à 20 heures.

13. Méthode telle que revendiquée dans la revendication 8 ou 9, dans laquelle la réaction d'époxydation est réalisé en la présence d'un catalyseur à une température de 20 à 220 °C, préférentiellement de 30 à 160 °C, plus préférentiellement de 50 à 130 C ; et/ou le rapport molaire entre le propylène et l'hydroperoxyde d'éthylbenzène dans la charge est 2:1-10:1, préférentiellement 3:1-8:1 ; la pression de la réaction d'époxydation est telle que le système de réaction est maintenu dans un état de phase entièrement liquide, et la pression est préférentiellement de 5 à 70 bars G, préférentiellement de 30 à 60 bars G.

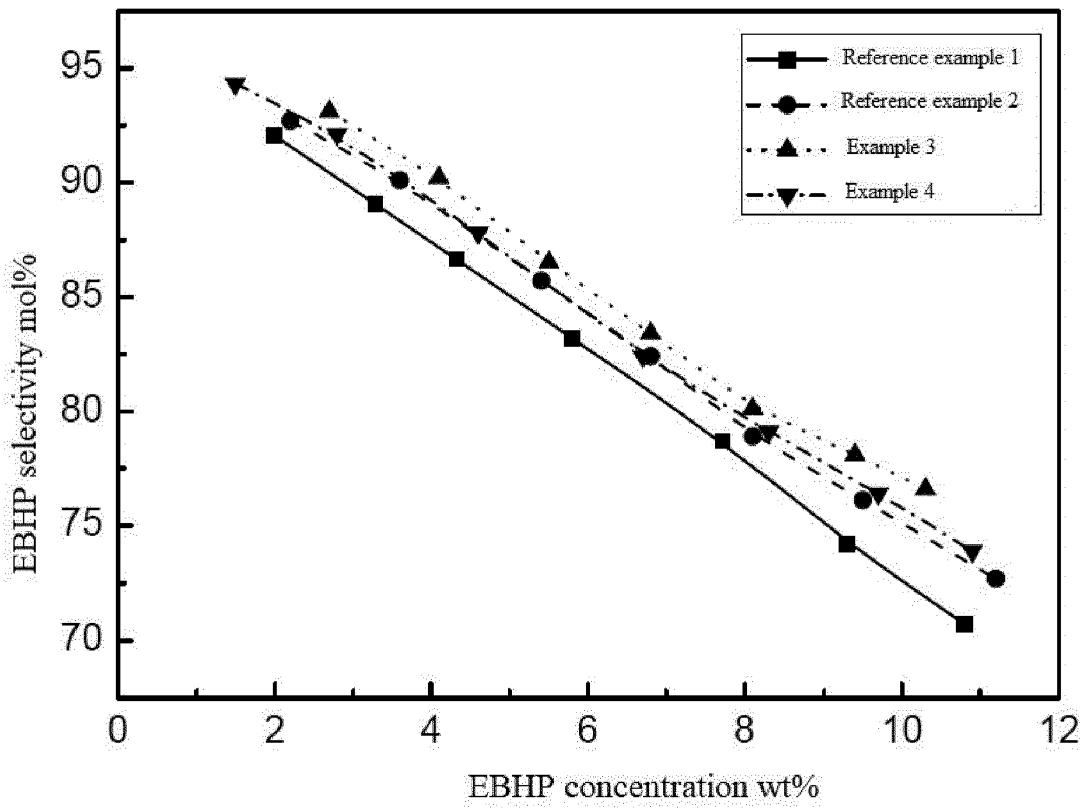
95
90
85
80
75
70
EBHP selectivity mol%
0
2
4
6
8
10
12
EBHP concentration wt%
Reference example 1
Reference example 2
Example 3
Example 4

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2798096 A
- US 3816540 A
- US 2820832 A
- US 4262143 A
- WO 2015101641 A1
- CN 201610021367
- CN 201510639697
- CN 201610601021
- CN 106334583 A

### Non-patent literature cited in the description

- **POKORSKA, Z ; RISZER, R ; SPADLO, M.** *Liquid phase oxidation of ethylbenzene by molecular oxygen to obtain the hydroperoxide,* 01 December 1973